# EUROPEAN PATENT SPECIFICATION

(11) **EP 3 738 582 B1**
(45) Date of publication and mention of the grant of the patent: **18.11.2026**
(21) Application number: 18900355.1
(22) Date of filing: 27.11.2018
(51) Int. Cl.: A61K 47/14, A61K 31/18, A61K 31/145, A61K 31/58, A61K 47/44, A61P 13/08, A61K 9/14, A61K 9/16

(54) **SOLID PARTICLE, PREPARATION METHOD THEREFOR, AND PHARMACEUTICAL COMPOSITION CONTAINING SOLID PARTICLE**
FESTES TEILCHEN, VERFAHREN ZU SEINER HERSTELLUNG UND PHARMAZEUTISCHE ZUSAMMENSETZUNG DAMIT
PARTICULE SOLIDE, SON PROCÉDÉ DE PRÉPARATION ET COMPOSITION PHARMACEUTIQUE CONTENANT DES PARTICULES SOLIDES

(30) Priority: 10.01.2018 CN 201810023696
(43) Date of publication of application: 18.11.2020
(73) Proprietor: Shanghai WD Pharmaceutical Co., Ltd, Shanghai 201203 (CN)
(72) Inventor: DONG, Liang Chang, Shanghai 201203 (CN); MA, Wenbo, Shanghai 201203 (CN); WANG, Lin, Shanghai 201203 (CN)
(74) Representative: Hoffmann Eitle
(86) International application number: PCT/CN2018/117622
(87) International publication number: WO 2019/137103

(56) References cited:
- EP-A1- 2 468 262
- WO-A1-03/063835
- WO-A1-2010/092596
- WO-A1-2010/111397
- WO-A1-2013/074205
- CN-A- 1 984 644
- CN-A- 101 237 891
- CN-A- 102 413 813
- CN-A- 104 039 311

## Description

The present application claims the priority of Chinese patent application No. CN201810023696.8, filed on January 10th, 2018.

### Field of invention

The present invention relates to a solid microparticle, a preparation method therefor, a pharmaceutical composition containing the same and the solid microparticle or the pharmaceutical composition for use as a medicament for treating benign prostatic hyperplasia.

### Prior arts

Avodart^{®} soft gelatin capsules manufactured by Glaxo Smithkline Pharmaceuticals are used for treating benign prostatic hyperplasia (BPH) of men's enlarged prostate. Avodart^{®}'s active ingredient is dutasteride. Dutasteride is a selective steroid 5α-reductase inhibitor, and 5α-reductase is an endoenzyme that can convert testosterone to DHT. Dutasteride has a chemical name of (5α,17β)-*N*-{2,5-bis(trifluoromethyl)phenyl}-3-oxo-4-azaandrost-1-ene-17-carboxamide, the empirical formula of which is C₂₇H₃₀F₆N₂O₂, molecular weight of which is 528.5, and the structural formula of which is shown as follows:

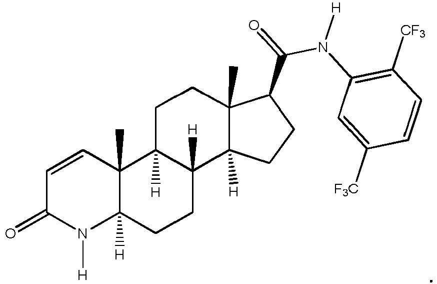

Dutasteride is a white to pale yellow powder with a melting point of 242°C to 250°C. It is soluble in ethanol (44 mg/mL), methanol (64 mg/mL), and polyethylene glycol 400 (3 mg/mL), but it is insoluble in water.

Hydrophobic active pharmaceutical ingredients (active pharmaceutical agents, APIs), such as dutasteride, have low bioavailability due to their poor solubility in an aqueous medium such as the gastrointestinal tract and solubilization techniques are commonly used to solve this problem. Commercially available Avodart^{®} soft gelatin capsule contains 0.5 mg dutasteride, which is dissolved in a mixture of caprylic/capric mono-/di-glyceride and dibutylhydroxytoluene.

U.S. patent application 20130052263 discloses a formula capable of increasing the solubility of hydrophobic compounds including dutasteride.

The manufacturing process of soft capsules is complicated and with high cost and low efficiency. In addition, soft capsules may be compromised in their integrity or lead to delayed dissolution during storage. The main reason for this phenomenon is the crosslinking and aging of the gelatin of the capsule shells, which forms a large network structure, thereby causing the soft capsule to rely on pepsin environment, otherwise pharmaceutical absorption will be affected. Therefore, U.S. Patent 9,622,981 discloses a liquid-filled hard gelatin capsule formulation that can reduce the manufacturing cost of soft capsules while maintain the bioavailability and stability of the soft gel dosage form. However, the liquid-filled hard capsules require a sealing step to prevent potential liquid leakage during manufacturing and storing period. In addition, the moisture in the hard capsules will migrate to the filled liquid formulation, resulting in a decrease in the moisture content of the hard capsules, which may cause the hard capsules to crack.

U.S. Patent 9,005,608 discloses a formulation for delivering the beneficial medicament Coenzyme Q (CoQ10), and the recrystallisation of dissolved CoQ10 can be prevented by incorporating a supersaturated self-emulsifying formula of CoQ10 into the pores of porous solid particles.

Another commercial product, Jalyn^{®} capsules, manufactured by Glaxo Smithkline Pharmaceuticals, is a pharmaceutical composition of dutasteride and tamsulosin, which can be used for treating benign prostatic hyperplasia (BPH). Tamsulosin is an α1A adrenergic receptor antagonist. Each Jalyn^{®} capsule contains dutasteride soft gelatin capsules and tamsulosin hydrochloride pills, and the capsules and pills are encapsulated in hard shell capsules of # 00 size. Although the combined product showed better treatment efficacy than the individual products, the # 00 size of Jalyn^{®} capsules reached the maximum value recommended by the FDA. Therefore, they are difficult to be swallowed by BPH patients, especially elderly patients.

WO 2010/111397 discloses particles comprising a porous carrier (e.g. dibasic calcium phosphate) and a liquid composition. The liquid composition contains CoQiO and Vitamin E as active agents, mono-, di-glycerides of caprylic acid (capmul), polyoxyl castor oil (Cremophor) or Polysorbate as surfactant. The liquid is sorbed on the solid carrier and the particles are filled into a capsule.

Therefore, there is a need for a new oral dosage form that can accommodate the combination of dutasteride and tamsulosin in a hard capsule, which has a size significantly smaller than the Jalyn capsule, and are bioequivalent to Jalyn^{®} capsules.

### Content of the present invention

The technical problem to be solved by the present invention is to provide a solid microparticle, a preparation method therefor and a pharmaceutical composition containing the same to overcome the disadvantages of the hydrophobic active pharmaceutical ingredients (active pharmaceutical agents, APIs) in the prior art, such as dutasteride, etc., which have low bioavailability, large dosage form size, poor patient compliance, etc.. The solid microparticle can effectively improve solubility of APIs and dissolution rate of medicaments, and can effectively improve the problems of easy cross-linking of soft capsules of commercialized products Avodart^{®} and Jalyn^{®} and oversized dosage form of Jalyn^{®} simultaneously.

The present invention is set out in the appended set of claims. Specifically, the present invention provides a solid microparticle as defined in claims 1-11, provides a method for preparing the solid microparticle as defined in claim 12, provides a pharmaceutical composition as defined in claims 13-14, and provides the solid microparticle or the pharmaceutical composition for use as a medicament for treating benign prostatic hyperplasia as defined in claim 15.

The present invention provides a solid microparticle, which comprises porous solid particles and a non-aqueous liquid formula, the non-aqueous liquid formula comprises 0.10% to 4.00% hydrophobic active pharmaceutical ingredient, 28.00% to 99.90% hydrophobic liquid solubilizer, 0 to 70.00% nonionic surfactant, and 0 to 1.00% antioxidant, the percentages refer to the weight percentages accounting for the non-aqueous liquid formula; and the hydrophobic liquid solubilizer comprises medium-chain mono-/di- glyceride, and/or propylene glycol fatty acid monoester.

In the present invention, the content of the hydrophobic active pharmaceutical ingredient may be 0.10% to 2.00% or 0.18% to 2.00%, and the percentages refer to the weight percentages accounting for the non-aqueous liquid formula.

In the present invention, the content of the hydrophobic active pharmaceutical ingredient may be 0.18%, 0.83%, 0.84%, 0.91%, 0.93%, 0.99%, 1.00%, 1.08%, 1.09%, 1.23%, 1.25%, 1.64%, 1.67%, 1.96% or 2.00%, and the percentages refer to the weight percentages accounting for the non-aqueous liquid formula.

In the present invention, the content of the hydrophobic liquid solubilizer may be 32.50% to 99.07%, 39.60% to 99.01%, or 49.50% to 74.26%, and the percentages refer to the weight percentages accounting for the non-aqueous liquid formula.

In the present invention, the content of the hydrophobic liquid solubilizer may be 32.50%, 39.60%, 46.00%, 47.06%, 49.02%, 49.18%, 49.50%, 59.41%, 59.87%, 60.67%, 60.83%, 61.73%, 63.00%, 65.57%, 72.55%, 73.51%, 74.26%, 75.00%, 82.16%, 82.43%, 83.33%, 98.27%, 98.79%, 99.01% or 99.07%, and the percentages refer to the weight percentages accounting for the non-aqueous liquid formula.

In the present invention, when the content of the nonionic surfactant is not 0, the content of the nonionic surfactant may be 15.00% to 66.67%, 24.51% to 59.41%, or 24.75% to 49.50%, and the percentages refer to the weight percentages accounting for the non-aqueous liquid formula.

In the present invention, when the content of the nonionic surfactant is not 0, the content of the nonionic surfactant may be 15.00%, 16.43%, 16.49%, 23.75%, 24.51%, 24.75%, 27.27%, 32.79%, 36.00%, 37.04%, 38.33%, 38.79%, 39.60%, 48.99%, 49.18%, 49.50%, 50.98%, 52.00%, 59.41% or 66.67%, and the percentages refer to the weight percentages accounting for the non-aqueous liquid formula.

In the present invention, when the content of the antioxidant is not 0, the content of the antioxidant may be 0.10% to 1.00% or 0.17% to 0.83%, and the percentages refer to the weight percentages accounting for the non-aqueous liquid formula.

In the present invention, when the content of the antioxidant is not 0, the content of the antioxidant may be 0.17%, 0.28%, 0.33%, 0.51%, 0.83%, or 1.00%, and the percentages refer to the weight percentages accounting for the non-aqueous liquid formula.

In a preferred embodiment of the present invention, the non-aqueous liquid formula comprises 0.10% to 2.00% hydrophobic active pharmaceutical ingredient, 97.00% to 99.80% medium-chain mono-/di- glyceride, and 0.10% to 1.00% antioxidant, and the percentages refer to the weight percentages accounting for the non-aqueous liquid formula.

In a preferred embodiment of the present invention, the non-aqueous liquid formula comprises 0.10% to 4.00% hydrophobic active pharmaceutical ingredient, 95.00% to 99.90% propylene glycol fatty acid monoester, and 0 to 1.00% antioxidant, and the percentages refer to the weight percentages accounting for the non-aqueous liquid formula.

In a preferred embodiment of the present invention, the weight ratio of the porous solid particle to the non-aqueous liquid formula may be a conventional ratio in the art, preferably 2: 1 to 3: 1, and more preferably 2: 1.

In a preferred embodiment of the present invention, the solid microparticle comprises 0.025% to 3.00% hydrophobic active pharmaceutical ingredient, 5.00% to 59.90% hydrophobic liquid solubilizer, and 0 to 45.00% nonionic surfactant, 0 to 0.60% antioxidant, and 40.00% to 75.00% porous solid particle, and the percentages refers to the weight percentages accounting for the solid microparticle.

In the present invention, the content of the hydrophobic active pharmaceutical ingredient may be 0.10% to 2.00%, such as 0.33%, 0.10% or 0.5%, and the percentages refer to the weight percentages accounting for the solid microparticle.

In the present invention, the content of the hydrophobic liquid solubilizer may be 5.00% to 39.90%, 11.50% to 35.00% or 13.20% to 33.00%, and the percentages refer to the weight percentages accounting for the solid microparticle.

In the present invention, the content of the hydrophobic liquid solubilizer may be 11.50%, 12.00%, 13.20%, 15.00%, 16.34%, 16.50%, 20.00%, 24.50%, 24.75%, 25.00%, 30.00%, 31.50%, 33.00%, 35.00%, 35.50%, 35.70%, 36.40%, 36.50% or 39.90%, and the percentages refer to the weight percentages accounting for the solid microparticle.

In the present invention, when the hydrophobic liquid solubilizer is medium-chain mono-/di-glyceride, the content of the hydrophobic active pharmaceutical ingredient is preferably 0.025% to 1.20%, and the content of the medium-chain mono-/di-glyceride is 24.50% to 59.90%, for example, 25.00% or 30.00%, and the percentages refer to the weight percentages accounting for the solid microparticle.

In the present invention, when the hydrophobic liquid solubilizer is propylene glycol fatty acid monoester, the content of the hydrophobic active pharmaceutical ingredient is preferably 0.025% to 2.40%, and the content of the propylene glycol fatty acid monoester is preferably 23.00% to 59.90%, such as 24.50%, 24.75%, 25.00%, 30.00%, 31.50%, 33.00%, 35.00%, 35.50%, 35.70%, 36.40%, 36.50% or 39.90%, and the percentages refer to the weight percentages account for the solid microparticle.

In the present invention, the content of the nonionic surfactant may be 0 to 40.00%. When the content of the nonionic surfactant is not 0, the content of the nonionic surfactant may be 4.50% to 40.00%, 8.17% to 19.80%, or 8.25% to 16.50%, and the percentages refer to the weight percentages accounting for the solid microparticle.

In the present invention, when the content of the nonionic surfactant is not 0, the content of the nonionic surfactant may be 4.50%, 5.00%, 8.17%, 8.25%, 9.50%, 10.00%, 13.00%, 15.00%, 16.33%, 16.50%, 18.00%, 19.80%, 20.00%, 23.00% or 40.00%, and the percentages refer to the weight percentages accounting for the solid microparticle.

In the present invention, when the content of the antioxidant is not 0, the content of the antioxidant may be 0.025% to 0.60%, such as 0.10%, 0.30%, or 0.33%, and the percentages refer to the weight percentages accounting for the solid microparticle.

In the present invention, the content of the porous solid particles may be 40.00% to 75.00% or 60.00% to 70.00%, and the percentages refer to the weight percentages accounting for the solid microparticle.

In the present invention, the content of the porous solid particles may be 40.00%, 40.70%, 45.00%, 49.50%, 50.00%, 59.50%, 60.00%, 63.67%, 64.57%, 64.67%, 66.67%, 69.5%, 69.57%, 69.67%, 70.00%, 74.50% or 75.00%, and the percentages refer to the weight percentages accounting for the solid microparticle.

In the present invention, the content of the non-aqueous liquid formula may be 25.00% to 60.00%, and the percentages refer to the weight percentages accounting for the solid microparticle.

In the present invention, the content of the non-aqueous liquid formula may be 25.00%, 25.50%, 30.00%, 30.33%, 30.43%, 30.50%, 33.33%, 35.33%, 35.43%, 36.33%, 40.00%, 40.50%, 50.00%, 50.50%, 55.00%, 59.30% or 60.00%, and the percentages refer to the weight percentages accounting for the solid microparticle.

In the present invention, the hydrophobic active pharmaceutical ingredient is dutasteride.

In the present invention, the medium-chain mono-/di-glyceride refers to an ester chain formed by the esterification of glycerol with one or two C₆-C₁₂ fatty acids. Commercially available medium-chain mono-/di- glyceride may comprises Capmul^{@} products manufactured by Abitec.

In the present invention, when the hydrophobic liquid solubilizer comprises medium-chain mono-/di- glyceride, the medium-chain of the medium-chain mono-/diglyceride is preferably a C₈-C₁₀ fatty acid chain. The medium-chain mono-/diglyceride is preferably mono-/di- caprylate/caprate (Capmul MCM), such as caprylic capric mono-/di- glyceride.

In the present invention, the propylene glycol fatty acid monoester refers to an ester chain formed by esterification of propylene glycol with a C₆-C₁₂ fatty acid.

In the present invention, when the hydrophobic liquid solubilizer comprises propylene glycol fatty acid monoester, the fatty acid of the propylene glycol fatty acid monoester is preferably a C₈-C₁₂ fatty acid chain. The propylene glycol fatty acid monoester is preferably propylene glycol monolaurate (type II) (LAUROGLYCOL 90) and/or propylene glycol monocaprylate (type II) (CAPRYOL 90), more preferably propylene glycol monocaprylate (type II) (CAPRYOL 90).

In the present invention, the type of the nonionic surfactant is preferably PEGylated type and/or polyol type, and more preferably PEGylated type.

Wherein, the PEGylated nonionic surfactant is preferably polyoxyethylene 35 castor oil (Kolliphor^{®} ELP) and/or caprylocaproyl polyoxylglycerides (LABASOL ALF).

Wherein, polyol type nonionic surfactant is preferably polysorbate 80 (TM).

In the present invention, the antioxidant may be a conventional antioxidant in the art, preferably dibutylhydroxytoluene (BHT) and/or butylhydroxyanisole (BHA), and more preferably dibutylhydroxytoluene (BHT).

In the present invention, the porous solid particles is calcium hydrogen phosphate (Fujicalin).

In the present invention, preferably, the porous solid particles have a specific surface area of >30 m²/g, and adsorb at least 0.40 mL/g the non-aqueous liquid formula, while maintaining flowability simultaneously.

In a preferred embodiment of the present invention, the solid microparticle comprise 0.0025% to 3.00% hydrophobic active pharmaceutical ingredient, 5.00% to 59.90% hydrophobic liquid solubilizer, 0 to 45.00% nonionic surfactant, 0.025% to 0.60% antioxidant, and 40.00% to 75.00% porous solid particles, and the percentages refers to the weight percentages accounting for the solid microparticle.

In a preferred embodiment of the present invention, the solid microparticle comprises 0.33% dutasteride, 33.00% propylene glycol monocaprylate (type II) (CAPRYOL 90), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 24.75% propylene glycol monocaprylate (type II) (CAPRYOL 90), 8.25% caprylocaproyl polyoxylglycerides (LABASOL ALF), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 16.5% propylene glycol monocaprylate (type II) (CAPRYOL 90), 16.5% caprylocaproyl polyoxylglycerides (LABASOL ALF), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 13.2% propylene glycol monocaprylate (type II) (CAPRYOL 90), 19.80% caprylocaproyl polyoxylglycerides (LABASOL ALF) and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 24.75% propylene glycol monocaprylate (type II) (CAPRYOL 90), 8.25% polyoxyethylene 35 castor oil (Kolliphor^{®} ELP), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 16.50% propylene glycol monocaprylate (type II) (CAPRYOL 90), 16.50% polyoxyethylene 35 castor oil (Kolliphor^{®} ELP), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 13.20% propylene glycol monocaprylate (type II) (CAPRYOL 90), 19.80% polyoxyethylene 35 castor oil (Kolliphor^{®} ELP), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 24.50% propylene glycol monocaprylate (type II) (CAPRYOL 90), 8.17% polyoxyethylene 35 castor oil (Kolliphor^{®} ELP), 0.33% dibutylhydroxytoluene (BHT), and 66.67% calcium hydrogen phosphate (Fujicalin), and the percentages refers to the weight percentages accounting for the solid microparticle.

In a preferred embodiment of the present invention, the solid microparticle comprises 0.33% dutasteride, 16.34% propylene glycol monocaprylate (type II) (CAPRYOL 90), 16.33% polyoxyethylene 35 castor oil (Kolliphor^{®} ELP), 0.33% dibutylhydroxytoluene (BHT), and 66.67% calcium hydrogen phosphate (Fujicalin), and the percentages refers to the weight percentages accounting for the solid particle.

The invention also provides a method for preparing the solid microparticle, which comprises the following steps:
(1) mixing the active pharmaceutical ingredient with the hydrophobic liquid solubilizer to obtain a solution when the solid microparticle does not comprise the nonionic surfactant and the antioxidant;
   or, mixing the nonionic surfactant and/or the antioxidant, and the active pharmaceutical ingredient and the hydrophobic liquid solubilizer to obtain solution A when the solid particle comprises the nonionic surfactant and/or the antioxidant;
(2) mixing the porous solid particles with the solution obtained in step (1);
or, mixing the porous solid particles with the solution A obtained in step (1).

In step (1), the mixing method may be a conventional mixing method in the art, and is preferably vortexing.

In step (2), the porous solid particles may further comprise a glidant. The glidant may be a conventional glidant in the art, and is preferably silicon dioxide.

The present invention also provides a pharmaceutical composition containing the solid microparticle and tamsulosin.

In the present invention, the pharmaceutical composition may be in a conventional dosage form, such as a hard capsule. The preparation method of the hard capsule can be a conventional preparation method in the art, and specifically, the solid microparticle and tamsulosin are filled into two-piece hard capsules.

In the present invention, the tamsulosin is a commercially available conventional product in the art, for example, it may be tamsulosin sustained-release pellets or tamsulosin hydrochloride sustained-release pellets.

In a preferred embodiment of the present invention, the pharmaceutical composition may also be used in combination, and the solid microparticle in the pharmaceutical composition are preferably dutasteride solid microparticle. The pharmaceutical composition and the tamsulosin sustained-release pellets can be encapsulated in two-piece hard capsules when they are used in combination, and the size of the hard capsule is not greater than # 0.

In the present invention, tamsulosin in the pharmaceutical composition can also be replaced with a conventional therapeutic medicament in the art, as long as the therapeutic medicament does not react with the solid microparticle in the present invention and affect the pharmacological activities of the medicament and the solid particle in the present invention.

In the present invention, the term "comprise ..." may also be expressed as "composed of".

For example, a solid microparticle comprises porous solid particle and a non-aqueous liquid formula. The non-aqueous liquid formula comprises 0.10% to 4.00% hydrophobic active pharmaceutical ingredient, 28.00% to 99.90% hydrophobic liquid solubilizer, and 0 to 70.00% nonionic surfactant, and 0 to 1.00% antioxidant. The percentages refer to the weight percentages, accounting for the non-aqueous liquid formula The hydrophobic liquid solubilizer comprises medium-chain mono-/diglyceride, and/or propylene glycol fatty acid monoester.

It can also be expressed as that a solid microparticle, which is composed of porous solid particle and a non-aqueous liquid formulation. The non-aqueous liquid formula is composed of 0.10% to 4.00% hydrophobic active pharmaceutical ingredient, 28.00% to 99.90% hydrophobic liquid solubilizer, and 0 to 70.00% nonionic surfactant, and 0 to 1.00% antioxidant. The percentages refer to the weight percentages accounting for the non-aqueous liquid formula. The hydrophobic liquid solubilizer is composed of medium-chain mono-/di- glyceride, and/or propylene glycol fatty acid monoester.

Without violating common knowledge in the art, the above-mentioned various preferred conditions can be optionally combined to obtain preferred embodiments of the present invention.

The reagents and raw materials used in the present invention are all commercially available.

The advantageous effects achieved by the present invention are as follows:
(1) The solid microparticle of the present invention improves the dissolving rate and dissolution of hydrophobic active pharmaceutical ingredients, such as dutasteride. The dutasteride hard capsule formulation prepared by the present invention has a dissolution about 11% to 30% faster than that of commercially available product Avodart within 0 to 30 minutes.
(2) The solid microparticle of the present invention is made into hard capsules, which can minimize the potential adsorption of hydrophobic active pharmaceutical ingredients, such as dutasteride, on porous solid particles in an aqueous medium with a pH of 3 or more without relying on the pepsin environment.
(3) The method for preparing the hard capsules of the present invention is simple, does not require the use of special equipment, and is suitable for large-scale industrial production. The dutasteride liquid solid microparticle in the present invention can be easily filled into two-piece hard capsules without the requirement of sealing.

Compared with liquid-filled hard gelatin capsules (U.S. Patent No. 9,622,981), it effectively solves the potential liquid leakage problem of the two-piece hard gelatin capsule sealed with a ribbon-like polymer.

Compared to soft gelatin capsules, the filling volume of the solid microparticle of the present invention is significantly smaller. In addition, the hard capsules of the present invention can also overcome the following disadvantages of soft gelatin capsules, including higher moisture content which is detrimental to moisture-sensitive products, additional impurities generated by migration of plasticizers to the fillers, and the manufacture process including three to seven days of tray drying.

### Brief description of the drawings

Fig.1 is a dutasteride dissolution curve figure of each formulation in embodiment 4 and the commercial product Avodart^{®} in a dissolution medium at pH = 3.

Fig.2 is a dutasteride dissolution curve figure of each formulation in embodiment 5 and the commercial product Avodart^{®} in a dissolution medium at pH = 3.

Fig.3 is a dutasteride dissolution curve figure of formulation in embodiment 6 and the commercial product Avodart^{®} in a dissolution medium without pepsin at pH=1.

Fig.4 is a dutasteride dissolution curve figure of formulation in embodiment 6 and the commercial product Avodart^{®} in a dissolution medium containing pepsin at pH=1.

### Detailed description of the preferred embodiment

The present invention is further described by the following embodiments. The experimental methods without indicating specific conditions are performed under conventional methods and conditions, or according to product specifications.

### Embodiment 1

The hydrophobic liquid solubilizers were mixed with Fujicalin at a weight ratio of 2: 1, and the flowability of the solid microparticles were estimated by measuring the tap density and bulk density of the solid microparticles.

The compression index (C.I.) and Hausner ratio (H.R.) could be calculated according to the following formula. $C . I . = 100 \times \left(\left({V}_{0}-{V}_{f}\right)/{V}_{0}\right)$ $H . R . = {V}_{0} / {V}_{f}$

Wherein *V*₀ and *V_{f}* are the bulk density and the tap density of the solid microparticles, respectively.

U.S. Pharmacopeia Chapter 39 <1174 "Powder Flow"> shows the correlation between the powder flowability with the compressibility index and the Hausner ratio (Table 1).

**Table 1: Flowability and the corresponding compressibility index and Hausner ratio**

| Compressibility index (%) | Flowability | Hausner ratio |
|---|---|---|
| ≤ 10 | Excellent | 1.00-1.11 |
| 11-15 | Good | 1.12-1.18 |
| 16-20 | Fair, no assistance needed | 1.19-1.25 |
| 21-15 | Passable, may shake | 1.26-1.34 |
| 26-31 | Poor-must shake, vibrate | 1.35-1.45 |
| 32-37 | Very poor | 1.46-1.59 |
| >38 | Very,very poor | >1.60 |

The measurement results are shown in Table 2. All hydrophobic liquid solubilizers, including propylene glycol monolaurate (type II) (LAUROGLYCOL 90), propylene glycol monocaprylate (type II) (CAPRYOL 90), caprylic capric mono-/diglyceride (type I) (Capmul MCM), etc., showed excellent flowability.

**Table 2: Flowability of the solid microparticles**

| No. | Hydrophobic | Weight | Silicon | Bulk | Tap | comp | Hausner |
|---|---|---|---|---|---|---|---|
| | liquid solubilizer | ratio of Fujicali n/non-aqueous liquid formula | dioxide (%) | density (g/mL) | density (g/mL) | ressib ility index | ratio |
| Embodiment 1-1 | Propylene glycol monolaurate (type II) (LAUROGLYCO L 90) | 2:1 | 0 | 0.685 | 0.748 | 8.50 | 1.09 |
| Embodiment 1-2 | Propylene glycol monocaprylate (type II) (CAPRYOL 90) | 2:1 | 0 | 0.690 | 0.771 | 10.5 | 1.12 |
| Embodiment 1-3 | Caprylic capric mono-/diglyceride (type I) (Capmul MCM) | 2:1 | 0 | 0.690 | 0.759 | 9.0 | 1.10 |

### Embodiment 2

Capryol 90 and Kolliphor^{®} ELP were mixed at a weight ratio of 1: 1, mixed with Fujicalin, and then mixed with silicon dioxide at different weight ratios. The flowability of the solid microparticles were estimated by measuring the tap density and the bulk density of the solid.

According to the measurement results shown in Table 3, silicon dioxide-free solid microparticles with a Fujicalin to non-aqueous liquid formula weight ratio of 2: 1 or 3: 1 had excellent flowability.

**Table 3: Flowability of the solid microparticles**

| No. | Weight ratio of Fujicalin/non-aqueous liquid formula | Silicon dioxide (%) | Bulk density (g/mL) | Tap density (g/mL) | compressibility index | Hausner ratio |
|---|---|---|---|---|---|---|
| Embodiment 2-1 Reference | 2:1 | 1.0 | 0.629 | 0.718 | 12.5 | 1.14 |
| Embodiment 2-2-Reference | 2:1 | 0.25 | 0.675 | 0.749 | 10.0 | 1.11 |
| Embodiment 2-3 | 2:1 | 0 | 0.696 | 0.765 | 9.0 | 1.10 |
| Embodiment 2-4 | 3:1 | 0 | 0.627 | 0.681 | 8.0 | 1.09 |
| Comparative embodiment 1 | 1:0 | 0 | 0.479 | 0.529 | 9.5 | 1.10 |

### Embodiment 3

When the solid microparticles were prepared, the content of each component might be as shown in the following table, the percentages referred to the weight percentages accounting for solid microparticles.

**Table 4**

| No. | Hydrophobic active pharmaceutical agent | Hydrophobic liquid solubilizer | Nonionic surfactant | Antioxidant | Porous solid particles |
|---|---|---|---|---|---|
| Embodiment 3-1 | 0.33% dutasteride | 35.00% CAPRYOL 90 | / | / | 64.67% Fujicalin |
| Embodiment 3-2 | 0.33% dutasteride | 35.00% CAPRYOL 90 | / | 0.10% BHT | 64.57% Fujicalin |
| Embodiment 3-3 | 0.33% dutasteride | 35.7% CAPRYOL 90 | / | 0.30% BHT | 63.67% Fujicalin |
| Embodiment 3-4-Reference | 0.33% dutasteride | 35.00% CAPRYOL 90 | / | 0.10% BHT | 34.57% Fujicalin and 30% silicon dioxide |
| Embodiment 3-5 | 0.33% dutasteride | 25.00% CAPRYOL 90 | 5.00% Kolliphor^{®}ELP | / | 69.67% Fujicalin |
| Embodiment 3-6 | 0.33% dutasteride | 25.00% CAPRYOL 90 | 5.00% LABASOL ALF | / | 69.67% Fujicalin |
| Embodiment 3-7 | 0.33% dutasteride | 25.00% CAPRYOL 90 | 5.00% Polysorbate 80 TM | / | 69.67% Fujicalin |
| Embodiment 3-8 | 0.33% dutasteride | 25.00% CAPRYOL 90 | 5.00% Kolliphor^{®}ELP | 0.10% BHT | 69.57% Fujicalin |
| Embodiment 3-9 | 0.33% dutasteride | 25.00% CAPRYOL 90 | 5.00% LABASOL ALF | 0.10% BHT | 69.57% Fujicalin |
| Embodiment 3-10 | 0.33% dutasteride | 25.00% CAPRYOL 90 | 5.00% Polysorbate 80 TM | 0.10% BHT | 69.57% Fujicalin |
| Embodiment 3-11-Reference | 0.33% dutasteride | 25.00% CAPRYOL 90 | 5.00% Polysorbate 80 TM | 0.10% BHT | 69.57% Neusilin |
| Embodiment 3-12 | 0.10% dutasteride | 39.90% CAPRYOL 90 | 15.00% Polysorbate 80 TM | / | 45.00% Fujicalin |
| Embodiment 3-13 | 0.50% dutasteride | 20.00% CAPRYOL 90 | 10.00% Polysorbate 80 TM | / | 69.50% Fujicalin |
| Embodiment 3-14 | 0.50% dutasteride | 11.50% CAPRYOL 90 | 13.00% Polysorbate 80 TM | / | 75.00% Fujicalin |
| Embodiment 3-15 | 0.50% dutasteride | 25.00% Capmul MCM | 4.50% Kolliphor^{®}ELP | / | 70.00% Fujicalin |
| Embodiment 3-16 | 0.50% dutasteride | 12.00% CAPRYOL 90 | 13.00% Kolliphor^{®}ELP | / | 74.50% Fujicalin |
| Embodiment 3-17 | 0.50% dutasteride | 15.00% CAPRYOL 90 | 15.00% Kolliphor^{®}ELP | / | 69.50% Fujicalin |
| Embodiment 3-18 | 0.50% dutasteride | 25.00% CAPRYOL 90 | 15.00% Kolliphor^{®}ELP | / | 59.50% Fujicalin |
| Embodiment 3-19 | 0.50% dutasteride | 30.00% CAPRYOL 90 | 20.00% Kolliphor^{®}ELP | / | 49.50% Fujicalin |
| Embodiment 3-20 | 0.50% dutasteride | 19.50% CAPRYOL 90 | 40.00% Kolliphor^{®}ELP | / | 40.00% Fujicalin |
| Embodiment 3-21 | 0.50% dutasteride | 30.00% Capmul MCM | 9.50% Kolliphor^{®}ELP | / | 60.00% Fujicalin |
| Embodiment 3-22 | 0.50% dutasteride | 31.50% LAUROGLYC OL 90 | 18.00% LABASOL ALF | / | 50.00% Fujicalin |
| Embodiment 3-23 | 0.50% dutasteride | 36.50% LAUROGLYC OL 90 | 23.00% LABASOL ALF | / | 40.00% Fujicalin |
| Embodiment 3-24 | 0.50% dutasteride | 36.40% LAUROGLYC OL 90 | 23.00% LABASOL ALF | 0.10% BHT | 40.00% Fujicalin |
| Embodiment 3-25 | 0.50% dutasteride | 35.50% LAUROGLYC OL 90 | 23.00% LABASOL ALF | 0.30% BHT | 40.70% Fujicalin |

The obtained solid microparticles with dutasteride solid microparticle formulas shown in Table 4 had a Hausner ratio of 1.00 to 1.18 and good flowability.

The obtained solid microparticles with dutasteride solid microparticle formulas shown in Table 4 were made into hard capsule formulations according to the preparation method of embodiment 4, and the dissolution thereof were detected according to the detection method in embodiment 4. They had about the same dissolutions as that of the formulation A-2, and could release about 18% to 25% faster than the reference listed formulation Avodart within 0-15 minutes.

### Embodiment 4

Dutasteride solid microparticle hard capsules were prepared, the formulations of the Dutasteride were shown as follows:

**Table 5: Compositions of the hard capsules in embodiment 4**

| / | Formulation AB | | Formulation A-1 | | Formulation A-2 | | Formulation A-3 | |
|---|---|---|---|---|---|---|---|---|
| Component | wt% | wt/ capsule mg | wt% | wt/ capsule mg | wt% | wt/ capsule mg | wt% | wt/capsule mg |
| Dutasteride | 0.33 | 0.50 | 0.33 | 0.50 | 0.33 | 0.50 | 0.33 | 0.50 |
| CAPRYOL 90 | 33.00 | 50.00 | 24.75 | 37.50 | 16.50 | 25.00 | 13.20 | 20.00 |
| LABASOL ALF | / | / | 8.25 | 12.50 | 16.50 | 25.00 | 19.80 | 30.00 |
| Fujicalin | 66.67 | 101.00 | 66.67 | 101.00 | 66.67 | 101.00 | 66.67 | 101.00 |
| Total | 100.00 | 151.50 | 100.00 | 151.50 | 100.00 | 151.50 | 100.00 | 151.50 |

### Preparation process of the capsules:

(1) prescribed amounts of dutasteride and CAPRYOL 90 were mixed and vortexed for 5 minutes;
(2) a prescribed amount of surfactant LABASOL ALF was added to obtain a clear solution;
(3) the solution was mixed with porous particles to obtain solid microparticles capable of flowing freely;
(4) 151mg solid microparticles were filled into # 2 hard capsules. Each capsule contained 0.5mg dutasteride.

### Measurement of dissolution:

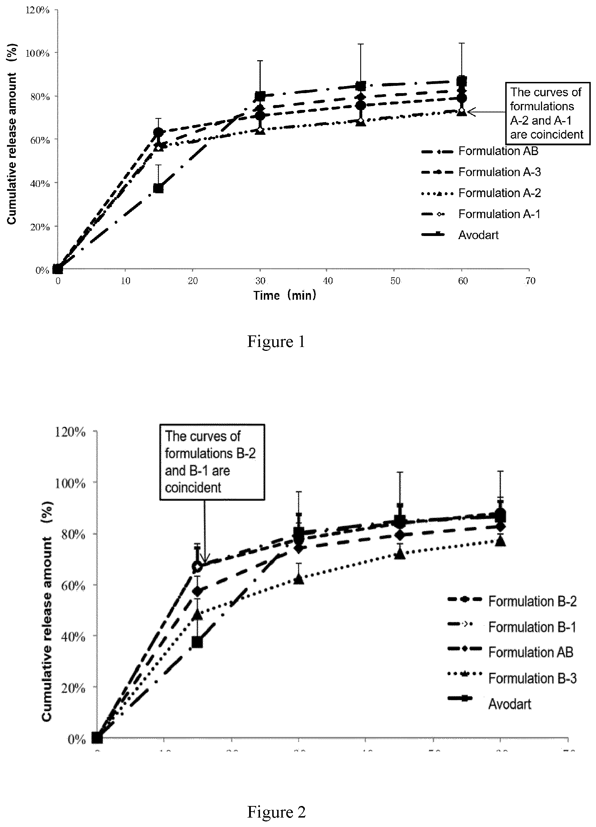

The obtained hard capsules of each formula in this embodiment and commercially available Avodart^{®} were stirred at 75 rpm according to USP II (paddle method) with 900mL 0.001N HCl, 1% cetyltrimethylammonium bromide (CTAB) solution as a dissolution medium at a temperature of 3710.5°C, and sampled at 15 min, 30 min, 45 min, and 60 min. The obtained sample solutions were filtered with a 0.45µm filter membrane, and quantitatively detected by an HPLC method. The dissolution of dutasteride were calculated. The chromatographic conditions are shown in Table 6, and the dissolution data are shown in Fig. 1.

**Table 6: HPLC chromatographic conditions**

| Instrument |
|---|
| Waters LC aqueous liquid chromatography with automatic sampling, injection and UV detection |

| Column |
|---|
| Thermo AQUASIL C18 0.46*150mm, 5µm |

| Operation parameters: |
|---|
| Mobile phase: acetonitrile, methanol and water (40: 20: 35) |
| Injection volume: 100 µL |
| Flow rate: 2.0 mL/min |
| Column temperature: 35°C |
| Detector wavelength: UV 240 nm |

Conclusion: the formulations AB, A-1, A-2 and A-3 of the present invention could release about 18% to 25% faster than the reference listed formulation Avodart within 0-15 minutes.

### Embodiment 5

The formulas of dutasteride formulations are shown as follows:

**Table 7: compositions of hard capsules in embodiment 5**

| / | Formulation B-1 | | Formulation B-2 | | Formulation B-3 | |
|---|---|---|---|---|---|---|
| Component | wt% | wt/capsule mg | wt% | wt/capsule mg | wt% | wt/capsule mg |
| Dutasteride | 0.33 | 0.50 | 0.33 | 0.50 | 0.33 | 0.50 |
| CAPRYOL 90 | 24.75 | 37.50 | 16.50 | 25.00 | 13.20 | 20.00 |
| Kolliphor^{®}ELP | 8.25 | 12.50 | 16.50 | 25.00 | 19.80 | 30.00 |
| Fujicalin | 66.67 | 101.00 | 66.67 | 101.00 | 66.67 | 101.00 |
| Total | 100.00 | 151.50 | 100.00 | 151.50 | 100.00 | 151.50 |

The preparation process and dissolution rate detection were the same as those in embodiment 4. The dissolution data are shown in Fig. 2.

Conclusions: the formulations B-1, B-2 and B-3 of the present invention could release about 11% to 30% faster than that of the reference listed formulation Avodart within 0 to15min, and the formulations B-1 and B-2 could release a little faster than Avodart within 0 to 60min.

### Embodiment 6

The formulation B-2 obtained in embodiment 5 and commercially available Avodart^{®} were stirred at 75 rpm according to USP II (paddle method) with 900mL 0.1N HCl, 1% cetyltrimethylammonium bromide (CTAB) solution as a dissolution medium at a temperature of 37±0.5°C. The dissolution medium might or might not contain 0.40% pepsin.

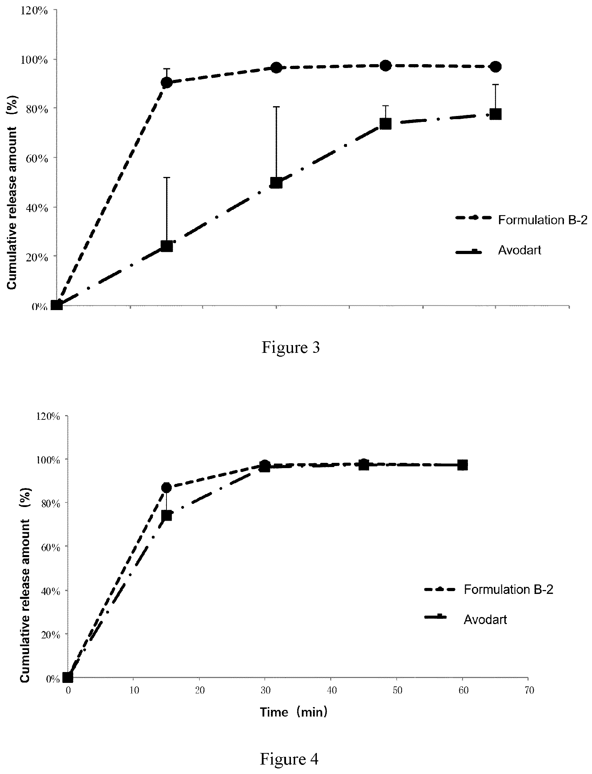

The detection method was the same as that in embodiment 4. The dissolution data are shown in Fig. 3 and 4.

### Conclusions:

Fig. 3 showed that the formulation B-2 obtained in the embodiment 5 had faster release rate and cumulative dissolution than Avodart^{®} in the dissolution medium without pepsin.

Fig. 4 showed that the formulation B-2 obtained in the embodiment 5 had about the same dissolution curves of hard capsules and soft capsules as those of Avodart^{®} in the dissolution medium containing pepsin.

Compared with Avodart^{®} gelatin soft capsules, dutasteride in the hard capsules of the formulation B-2 showed more consistent dissolution rates without being influenced by the presence of pepsin in the dissolution medium, which might be caused by the potential cross-linking of the Avodart^{®} soft gelatin capsules, resulting in that the dissolution of the medicament depended on pepsin.

### Embodiment 7- Reference

According to the formula and preparation method of embodiment 5, 171.6 mg tamsulosin sustained-release pills and 150 mg dutasteride solid microparticles (formulation B-2) were prepared and filled together into 0 # hard gelatin capsules. Each capsule contained 0.4 mg tamsulosin and 0.5 mg dutasteride.

### Embodiment 8

The formulations of the dutasteride were shown as follows:

**Table 8: Compositions of hard capsules in embodiment 8**

| | Formulation B-4 | | Formulation B-5 | |
|---|---|---|---|---|
| Component | wt% | wt/capsule mg | wt% | wt/ capsule mg |
| Dutasteride | 0.33 | 0.50 | 0.33 | 0.50 |
| CAPRYOL 90 | 24.50 | 37.12 | 16.34 | 24.76 |
| Kolliphor^{®}ELP | 8.17 | 12.38 | 16.33 | 24.74 |
| Fujicalin | 66.67 | 101.00 | 66.67 | 101.00 |
| BHT | 0.33 | 0.50 | 0.33 | 0.50 |
| Total | 100.00 | 151.50 | 100.00 | 151.50 |

The preparation process and detection of dissolution were the same as those in the embodiment 4.

The dissolution data of the formulation B-4 was similar to that of the formulation B-1 in the embodiment 5, and the dissolution data of the formulation B-5 was similar to that of the formulation B-2 in the embodiment 5.

### Comparative embodiment 2

The dutasteride solid microparticle hard capsules were prepared according to the formulas in the table below. The preparation process and detection method were the same as those in the embodiment 4. Captex^{®} 355 are triglycerides of caprylic acid (C₈) and capric acid (C₁₀).

**Table 9**

| Component | wt% | wt/capsule mg |
|---|---|---|
| Dutasteride | 0.33 | 0.50 |
| CAPRYOL 90 | 24.75 | 37.50 |
| Captex^{®}355 | 8.25 | 12.50 |
| Fujicalin | 66.67 | 101.00 |
| Total | 100.00 | 151.50 |

When the hydrophobic liquid solubilizer comprised medium-chain triglycerides, the self-emulsifying property of the prepared dutasteride solid microparticles were significantly lower than that of the dutasteride solid microparticles of the present application, and the dissolution rate thereof was also significantly lower than that of the dutasteride solid microparticles of the present application.

## Claims

1. A solid microparticle, which comprises porous solid particles and a non-aqueous liquid formula,
the non-aqueous liquid formula comprises 0.10% to 4.00% hydrophobic active pharmaceutical ingredient,
28.00% to 99.90% hydrophobic liquid solubilizer,
0 to 70.00% nonionic surfactant, and
0 to 1.00% antioxidant, the percentages refer to the weight percentages accounting for the non-aqueous liquid formula; wherein
the hydrophobic liquid solubilizer comprises medium-chain mono-/di- glyceride, and/or propylene glycol fatty acid monoester;
the hydrophobic active pharmaceutical ingredient is dutasteride, and
the porous solid particles are calcium hydrogen phosphate.

2. The solid microparticle as claimed in claim 1, wherein the content of the hydrophobic active pharmaceutical ingredient is 0.10% to 2.00% or 0.18% to 2.00%, and the percentages refer to the weight percentages accounting for the non-aqueous liquid formula;
and/or, the content of the hydrophobic liquid solubilizer is 32.50% to 99.07%, 39.60% to 99.01%, or 49.50% to 74.26%, and the percentages refer to the weight percentages accounting for the non-aqueous liquid formula;
and/or, when the content of the nonionic surfactant is not 0, the content of the nonionic surfactant is 15.00% to 66.67%, 24.51% to 59.41%, or 24.75% to 49.50%, and the percentages refer to the weight percentages accounting for the non-aqueous liquid formula;
and/or, when the content of the antioxidant is not 0, the content of the antioxidant is 0.10% to 1.00% or 0.17% to 0.83%, and the percentages refer to the weight percentages accounting for the non-aqueous liquid formula.

3. The solid microparticle as claimed in claim 1 or 2, wherein,
when the hydrophobic liquid solubilizer is medium-chain mono-/di- glyceride, the non-aqueous liquid formula comprises 0.10% to 2.00% hydrophobic active pharmaceutical ingredient, 97.00% to 99.80% medium-chain mono-/di- glyceride, and 0.10% to 1.00% antioxidant, and the percentages refer to the weight percentages accounting for the non-aqueous liquid formula;
and/or, when the hydrophobic liquid solubilizer is propylene glycol fatty acid monoester, the non-aqueous liquid formula comprises 0.10% to 4.00% hydrophobic active pharmaceutical ingredient, 95.00% to 99.90% propylene glycol fatty acid monoester, and 0 to 1.00% antioxidant, and the percentages refer to the weight percentages accounting for the non-aqueous liquid formula;
and/or, the weight ratio of the porous solid particles to the non-aqueous liquid formula is 2: 1 to 3: 1, preferably 2: 1.

4. The solid microparticle as claimed in at least one of claims 1 to 3, wherein the solid microparticle comprises 0.025% to 3.00% hydrophobic active pharmaceutical ingredient, 5.00% to 59.90% hydrophobic liquid solubilizer, and 0 to 45.00% nonionic surfactant, 0 to 0.60% antioxidant, and 40.00% to 75.00% porous solid particles, and the percentages refers to the weight percentages accounting for the solid microparticle.

5. The solid microparticle as claimed in claim 4, wherein the content of the hydrophobic active pharmaceutical ingredient is 0.10% to 2.00%, and the percentages refer to the weight percentages accounting for the solid microparticle;
and/or, the content of the hydrophobic liquid solubilizer is 5.00% to 39.90%, 11.50% to 35.00%, or 13.20% to 33.00%, and the percentages refer to the weight percentages accounting for the solid microparticle;
and/or, the content of the nonionic surfactant is 0 to 40.00%, 4.50% to 40.00%, 8.25% to 19.80%, or 8.25% to 16.50%, and the percentages refer to the weight percentages accounting for the solid microparticle;
and/or, the content of the antioxidant is 0.025% to 0.60%, and the percentages refer to the weight percentages accounting for the solid microparticle;
and/or, the content of the porous solid particles is 40.00% to 75.00% or 60.00% to 70.00%, and the percentages refer to the weight percentages accounting for the solid microparticle.

6. The solid microparticle as claimed in claim 4, wherein,
when the hydrophobic liquid solubilizer is medium-chain mono-/di- glyceride, the content of the hydrophobic active pharmaceutical ingredient is 0.025% to 1.20%, the content of medium-chain mono-/di- glyceride is 24.50% to 59.90%, and the percentages refer to the weight percentages accounting for the solid microparticle;
and/or, when the hydrophobic liquid solubilizer is propylene glycol fatty acid monoester, the content of the hydrophobic active pharmaceutical ingredient is 0.025% to 2.40%, the content of the propylene glycol fatty acid monoester is 23.00% to 59.90%, and the percentages refer to the weight percentages accounting for the solid microparticle;
and/or, the solid microparticle comprises 0.0025% to 3.00% hydrophobic active pharmaceutical ingredient, 5.00% to 59.90% hydrophobic liquid solubilizer, 0 to 45.00% nonionic surfactant, 0.025% to 0.60% antioxidant, and 40.00% to 75.00% porous solid particles, and the percentages refers to the weight percentages accounting for the solid microparticle.

7. The solid microparticle as claimed in at least one of claims 1 to 6, wherein,
when the hydrophobic liquid solubilizer comprises medium-chain mono-/diglyceride, the medium-chain mono-/di- glyceride is preferably mono-/di-caprylate/caprate, and more preferably caprylic capric mono-/di- glyceride;
and/or, when the hydrophobic liquid solubilizer comprises propylene glycol fatty acid monoester, the propylene glycol fatty acid monoester is preferably propylene glycol monolaurate type II and/or propylene glycol monocaprylate type II, and more preferably propylene glycol monocaprylate type II;
and/or, the type of the nonionic surfactant is PEGylated type and/or polyol type, preferably PEGylated type; and the PEGylated type nonionic surfactant is preferably polyoxyethylene 35 castor oil and/or caprylocaproyl polyoxylglycerides; and the polyol type nonionic surfactant is preferably polysorbate 80;
and/or, the antioxidant is dibutylhydroxytoluene and/or butylhydroxyanisole, preferably dibutylhydroxytoluene;
and/or, the porous solid particles have a specific surface area of >30 m²/g, and adsorb at least 0.40 mL/g the non-aqueous liquid formula while maintaining flowability simultaneously.

8. The solid microparticle as claimed in claim 7, wherein the solid microparticle comprises 0.33% dutasteride, 33.00% propylene glycol monocaprylate type II, and 66.67% calcium hydrogen phosphate, and the percentages refer to the weight percentages accounting for the solid microparticle;
or, the solid microparticle comprises 0.33% dutasteride, 24.75% propylene glycol monocaprylate type II, 8.25% caprylocaproyl polyoxylglycerides, and 66.67% calcium hydrogen phosphate, and the percentages refer to the weight percentages accounting for the solid microparticle;
or, the solid microparticle comprises 0.33% dutasteride, 16.50% propylene glycol monocaprylate type II, 16.50% caprylocaproyl polyoxylglycerides, and 66.67% calcium hydrogen phosphate, and the percentages refer to the weight percentages accounting for the solid microparticle;
or, the solid microparticle comprises 0.33% dutasteride, 13.20% propylene glycol monocaprylate type II, 19.80% caprylocaproyl polyoxylglycerides, and 66.67% calcium hydrogen phosphate, and the percentages refer to the weight percentages accounting for the solid microparticle;
or, the solid microparticle comprises 0.33% dutasteride, 24.75% propylene glycol monocaprylate type II, 8.25% polyoxyethylene 35 castor oil, and 66.67% calcium hydrogen phosphate, and the percentages refer to the weight percentages accounting for the solid microparticle;
or, the solid microparticle comprises 0.33% dutasteride, 16.50% propylene glycol monocaprylate type II, 16.50% polyoxyethylene 35 castor oil, and 66.67% calcium hydrogen phosphate, and the percentages refer to the weight percentages accounting for the solid microparticle;
or, the solid microparticle comprises 0.33% dutasteride, 13.20% propylene glycol monocaprylate type II, 19.80% polyoxyethylene 35 castor oil, and 66.67% calcium hydrogen phosphate, and the percentages refer to the weight percentages accounting for the solid microparticle;
or, the solid microparticle comprises 0.33% dutasteride, 24.50% propylene glycol monocaprylate, 8.17% polyoxyethylene 35 castor oil, 0.33% dibutylhydroxytoluene, and 66.67% calcium hydrogen phosphate, and the percentages refers to the weight percentages accounting for the solid microparticle;
or, the solid microparticle comprises 0.33% dutasteride, 16.34% propylene glycol monocaprylate, 16.33% polyoxyethylene 35 castor oil, 0.33% dibutylhydroxytoluene, and 66.67% calcium hydrogen phosphate, and the percentages refers to the weight percentages accounting for the solid particle.

9. The solid microparticle as claimed in claim 1, wherein the content of the non-aqueous liquid formula is 25.00% to 60.00%, and the percentage refers to the weight percentages accounting for the solid microparticle.

10. The solid microparticle as claimed in claim 1, wherein the medium-chain mono-/di- glyceride is an ester chain formed by the esterification of glycerol with one or two C₆-C₁₂ fatty acids;
or, the propylene glycol fatty acid monoester is an ester chain formed by esterification of propylene glycol with a C₆-C₁₂ fatty acid.

11. The solid microparticle as claimed in claim 1, wherein when the hydrophobic liquid solubilizer comprises medium-chain mono-/di- glyceride, the medium-chain of the medium-chain mono-/di- glyceride is a C₈-C₁₀ fatty acid chain;
or, when the hydrophobic liquid solubilizer comprises propylene glycol fatty acid monoester, the fatty acid of the propylene glycol fatty acid monoester is a C₈-C₁₂ fatty acid chain.

12. A method for preparing the solid microparticle as claimed in at least one of claims 1 to 11, which comprises the following steps:
(1) mixing the pharmaceutical active ingredient with the hydrophobic liquid solubilizer to obtain a solution when the solid microparticle do not comprise the nonionic surfactant and the antioxidant;
or, mixing the nonionic surfactant and/or the antioxidant, and the pharmaceutical active ingredient and the hydrophobic liquid solubilizer to obtain solution A when the solid microparticle comprises the nonionic surfactant and/or the antioxidant;
and (2) mixing the porous solid particles with the solution obtained in step (1);
or, mixing the porous solid particles with the solution A obtained in step (1).

13. A pharmaceutical composition comprising the solid microparticle as claimed in at least one of claims 1 to 11 and tamsulosin.

14. The pharmaceutical composition as claimed in claim 13, wherein the pharmaceutical composition is in hard capsule form;
or, the tamsulosin is tamsulosin sustained-release pellets or tamsulosin hydrochloride sustained-release pellets.

15. The solid microparticle as claimed in at least one of claims 1-11 or the pharmaceutical composition as claimed in at least one of claims 13-14 for use as a medicament for treating benign prostatic hyperplasia, wherein the hydrophobic active pharmaceutical ingredient is dutasteride.

## Patentansprüche

1. Festes Mikroteilchen, das poröse feste Teilchen und eine nicht-wässrige flüssige Formulierung umfasst,
wobei die nicht-wässrige flüssige Formulierung umfasst: 0,10 % bis 4,00 % an hydrophobem pharmazeutischem Wirkstoff,
28,00 % bis 99,90 % an hydrophobem flüssigem Lösungsvermittler,
0 bis 70,00 % an nicht-ionischem Tensid, und
0 bis 1,00 % an Antioxidans, wobei sich die Prozente auf die Gewichtsprozente beziehen, die auf die nicht-wässrige flüssige Formulierung entfallen; wobei
der hydrophobe flüssige Lösungsvermittler mittelkettiges Mono-/Diglycerid und/oder Propylenglykolfettsäure-Monoester umfasst;
der hydrophobe pharmazeutische Wirkstoff Dutasterid ist, und
die porösen festen Teilchen Kalziumwasserstoffphosphat sind.

2. Festes Mikroteilchen nach Anspruch 1, wobei der Gehalt des hydrophoben pharmazeutischen Wirkstoffs 0,10 % bis 2,00 % oder 0,18 % bis 2,00 % beträgt, und sich die Prozente auf die Gewichtsprozente beziehen, die auf die nicht-wässrige flüssige Formulierung entfallen;
und/oder der Gehalt des hydrophoben flüssigen Lösungsvermittlers 32,50 % bis 99,07 %, 39,60 % bis 99,01 % oder 49,50 % bis 74,26 % beträgt und sich die Prozente auf die Gewichtsprozente beziehen, die auf die nicht-wässrige flüssige Formulierung entfallen;
und/oder, wenn der Gehalt des nicht-ionischen Tensids nicht 0 ist, der Gehalt des nicht-ionischen Tensids 15,00 % bis 66,67 %, 24,51 % bis 59,41 % oder 24,75 % bis 49,50 % beträgt und sich die Prozente auf die Gewichtsprozente beziehen, die auf die nicht-wässrige flüssige Formulierung entfallen;
und/oder, wenn der Gehalt des Antioxidans nicht 0 ist, der Gehalt des Antioxidans 0,10 % bis 1,00 % oder 0,17 % bis 0,83 % beträgt und sich die Prozente auf die Gewichtsprozente beziehen, die auf die nicht-wässrige flüssige Formulierung entfallen.

3. Festes Mikroteilchen nach Anspruch 1 oder 2, wobei,
wenn der hydrophobe flüssige Lösungsvermittler ein mittelkettiges Mono-/Diglycerid ist, die nicht-wässrige flüssige Formulierung 0,10 % bis 2,00 % an hydrophobem pharmazeutischem Wirkstoff, 97,00 % bis 99,80 % an mittelkettigem Mono-/Diglycerid und 0,10 % bis 1,00 % an Antioxidans umfasst und sich die Prozente auf die Gewichtsprozente beziehen, die auf die nicht-wässrige flüssige Formulierung entfallen;
und/oder, wenn der hydrophobe flüssige Lösungsvermittler Propylenglykolfettsäure-Monoester ist, die nicht wässrige flüssige Formulierung 0,10 % bis 4,00 % an hydrophobem pharmazeutischem Wirkstoff, 95,00 % bis 99,90 % an Propylenglykolfettsäure-Monoester und 0 bis 1,00 % an Antioxidans umfasst und sich die Prozente auf die Gewichtsprozente beziehen, die auf die nicht-wässrige flüssige Formulierung entfallen;
und/oder das Gewichtsverhältnis der porösen festen Teilchen zu der nicht-wässrigen flüssigen Formulierung 2:1 bis 3:1, bevorzugt 2:1 beträgt.

4. Festes Mikroteilchen nach mindestens einem der Ansprüche 1 bis 3, wobei das feste Mikroteilchen 0,025 % bis 3,00 % an hydrophobem pharmazeutischem Wirkstoff, 5,00 % bis 59,90 % an hydrophobem flüssigem Lösungsvermittler und 0 bis 45,00 % an nicht-ionischem Tensid, 0 bis 0,60 % an Antioxidans und 40,00 % bis 75,00 % an porösen festen Teilchen umfasst und sich die Prozente auf die Gewichtsprozente des festen Mikroteilchens beziehen.

5. Festes Mikroteilchen nach Anspruch 4, wobei der Gehalt des hydrophoben pharmazeutischen Wirkstoffs 0,10 % bis 2,00 % beträgt und sich die Prozente auf die Gewichtsprozente beziehen, die auf das feste Mikroteilchen entfallen;
und/oder der Gehalt des hydrophoben flüssigen Lösungsvermittlers 5,00 % bis 39,90 %, 11,50 % bis 35,00 % oder 13,20 % bis 33,00 % beträgt und sich die Prozente auf die Gewichtsprozente beziehen, die auf das feste Mikroteilchen entfallen;
und/oder der Gehalt des nicht-ionischen Tensids 0 bis 40,00 %, 4,50 % bis 40,00 %, 8,25 % bis 19,80 % oder 8,25 % bis 16,50 % beträgt und sich die Prozente auf die Gewichtsprozente beziehen, die auf das feste Mikroteilchen entfallen;
und/oder der Gehalt des Antioxidans 0,025 % bis 0,60 % beträgt und sich die Prozente auf die Gewichtsprozente beziehen, die auf das feste Mikroteilchen entfallen;
und/oder der Gehalt der porösen festen Teilchen 40,00 % bis 75,00 % oder 60,00 % bis 70,00 % beträgt und sich die Prozente auf die Gewichtsprozente beziehen, die auf das feste Mikroteilchen entfallen.

6. Festes Mikroteilchen nach Anspruch 4, wobei,
wenn der hydrophobe flüssige Lösungsvermittler ein mittelkettiges Mono-/Diglycerid ist, der Gehalt des hydrophoben pharmazeutischen Wirkstoffs 0,025 % bis 1,20 % beträgt, der Gehalt an mittelkettigem Mono-/Diglycerid 24,50 % bis 59,90 % beträgt und sich die Prozente auf die Gewichtsprozente beziehen, die auf das feste Mikroteilchen entfallen;
und/oder, wenn der hydrophobe flüssige Lösungsvermittler Propylenglykolfettsäure-Monoester ist, der Gehalt des hydrophoben pharmazeutischen Wirkstoffs 0,025 % bis 2,40 % beträgt, der Gehalt des Propylenglykolfettsäure-Monoesters 23,00 % bis 59,90 % beträgt und sich die Prozente auf die Gewichtsprozente des festen Mikroteilchens beziehen;
und/oder das feste Mikroteilchen 0,0025 % bis 3,00 % an hydrophobem pharmazeutischem Wirkstoff, 5,00 % bis 59,90 % an hydrophobem flüssigem Lösungsvermittler, 0 bis 45,00 % an nicht-ionischem Tensid, 0,025 % bis 0,60 % an Antioxidans und 40,00 % bis 75,00 % an porösen festen Teilchen umfasst und sich die Prozente auf die Gewichtsprozente beziehen, die auf das feste Mikroteilchen entfallen.

7. Festes Mikroteilchen nach mindestens einem der Ansprüche 1 bis 6, wobei,
wenn der hydrophobe flüssige Lösungsvermittler mittelkettiges Mono-/Diglycerid umfasst, das mittelkettige Mono-/Diglycerid bevorzugt Mono-/Dicaprylat/Caprat und bevorzugter Capryl-Caprinsäure-Mono-/Diglycerid ist;
und/oder, wenn der hydrophobe flüssige Lösungsvermittler Propylenglykolfettsäure-Monoester umfasst, der Propylenglykolfettsäure-Monoester bevorzugt Propylenglykolmonolaurat Typ II und/oder Propylenglykolmonocaprylat Typ II und bevorzugter Propylenglykolmonocaprylat Typ II ist;
und/oder der Typ des nicht-ionischen Tensids PEGylierter Typ und/oder Polyoltyp, bevorzugt PEGylierter Typ ist; und das nicht-ionische Tensid vom PEGylierten Typ bevorzugt Polyoxyethylen-35-Rizinöl und/oder Caprylocaproyl-Polyoxylglyceride ist; und das nicht-ionische Tensid vom Typ Polyol bevorzugt Polysorbat 80 ist;
und/oder das Antioxidans Dibutylhydroxytoluol und/oder Butylhydroxyanisol, bevorzugt Dibutylhydroxytoluol ist;
und/oder die porösen festen Teilchen eine spezifische Oberfläche von >30m²/g aufweisen und mindestens 0,40 ml/g der nicht-wässrigen flüssigen Formulierung adsorbieren, während gleichzeitig die Fließfähigkeit aufrechterhalten wird.

8. Festes Mikroteilchen nach Anspruch 7, wobei das feste Mikroteilchen 0,33 % an Dutasterid, 33,00 % an Propylenglykol-Monocaprylat Typ II und 66,67 % an Kalziumwasserstoffphosphat umfasst und sich die Prozentsätze auf die Gewichtsprozente beziehen, die auf das feste Mikroteilchen entfallen;
oder, das feste Mikroteilchen 0,33 % an Dutasterid, 24,75 % an Propylenglykol-Monocaprylat Typ II, 8,25 % an Caprylocaproylpolyoxylglyceriden und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 16,50 % an Propylenglykol-Monocaprylat Typ II, 16,50 % an Caprylocaproylpolyoxylglyceriden und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 13,20 % an Propylenglykol-Monocaprylat Typ II, 19,80 % an Caprylocaproylpolyoxylglyceriden und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 24,75 % an Propylenglykol-Monocaprylat Typ II, 8,25 % an Polyoxyethylen 35 Rizinusöl und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 16,50 % an Propylenglykol-Monocaprylat Typ II, 16,50 % an Polyoxyethylen 35 Rizinusöl und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 13,20 % an Propylenglykol-Monocaprylat Typ II, 19,80 % an Polyoxyethylen 35 Rizinusöl und 66,67 % an Calciumhydrogenphosphat umfasst und sich die Prozentsätze auf die Gewichtsprozente beziehen, die auf das feste Mikroteilchen entfallen;
oder, das feste Mikroteilchen 0,33 % an Dutasterid, 24,50 % an Propylenglykol-Monocaprylat, 8,17 % an Polyoxyethylen 35 Rizinusöl, 0,33 % an Dibutylhydroxytoluol und 66,67 % an Calciumhydrogenphosphat umfasst und sich die Prozente auf die Gewichtsprozente beziehen, die auf das feste Mikroteilchen entfallen;
oder, das feste Mikroteilchen 0,33 % an Dutasterid, 16,34 % an Propylenglykol-Monocaprylat, 16,33 % an Polyoxyethylen 35 Rizinusöl, 0,33 % an Dibutylhydroxytoluol und 66,67 % an Calciumhydrogenphosphat umfasst und sich die Prozente auf die Gewichtsprozente beziehen, die auf das feste Teilchen entfallen.

9. Festes Mikroteilchen nach Anspruch 1, wobei der Gehalt der nicht-wässrigen flüssigen Formulierung 25,00 % bis 60,00 % beträgt und sich die Prozente auf die Gewichtsprozente beziehen, die auf das feste Mikroteilchen entfallen.

10. Festes Mikroteilchen nach Anspruch 1, wobei das mittelkettige Mono-/Diglycerid eine Esterkette ist, die durch die Veresterung von Glycerin mit einer oder zwei C₆-C₁₂-Fettsäuren gebildet wird;
oder, der Propylenglykolfettsäure-Monoester eine Esterkette ist, die durch Veresterung von Propylenglykol mit einer C₆-C₁₂-Fettsäure gebildet wird.

11. Festes Mikroteilchen nach Anspruch 1, wobei, wenn der hydrophobe flüssige Lösungsvermittler ein mittelkettiges Mono-/Diglycerid umfasst, die Mittelkette des mittelkettigen Mono-/Diglycerids eine C₈-C₁₀-Fettsäurekette ist;
oder, wenn der hydrophobe flüssige Lösungsvermittler Propylenglykolfettsäure-Monoester umfasst, die Fettsäure des Propylenglykolfettsäure-Monoesters eine C₈-C₁₂-Fettsäurekette ist.

12. Verfahren zur Herstellung des festen Mikroteilchens nach mindestens einem der Ansprüche 1 bis 11, umfassend die folgenden Schritte:
(1) Mischen des pharmazeutischen Wirkstoffs mit dem hydrophoben flüssigen Lösungsvermittler, um eine Lösung zu erhalten, wenn das feste Mikroteilchen das nicht-ionische Tensid und das Antioxidans nicht umfasst;
oder Mischen des nicht-ionischen Tensids und/oder des Antioxidans und des pharmazeutischen Wirkstoffs und des hydrophoben flüssigen Lösungsvermittlers, um Lösung A zu erhalten, wenn das feste Mikroteilchen das nicht-ionische Tensid und/oder das Antioxidans umfasst;
und (2) Mischen der porösen festen Teilchen mit der in Schritt (1) erhaltenen Lösung;
oder Mischen der porösen festen Teilchen mit der in Schritt (1) erhaltenen Lösung A.

13. Pharmazeutische Zusammensetzung, umfassend das feste Mikroteilchen nach mindestens einem der Ansprüche 1 bis 11 und Tamsulosin.

14. Pharmazeutische Zusammensetzung nach Anspruch 13, wobei die pharmazeutische Zusammensetzung in Form einer harten Kapsel ist;
oder, das Tamsulosin Tamsulosin-Retardpellets oder Tamsulosin-Hydrochlorid-Retardpellets ist.

15. Festes Mikroteilchen nach mindestens einem der Ansprüche 1 bis 11 oder pharmazeutische Zusammensetzung nach mindestens einem der Ansprüche 13 bis 14 zur Verwendung als Arzneimittel zur Behandlung einer gutartigen Prostatahyperplasie, wobei der hydrophobe pharmazeutische Wirkstoff Dutasterid ist.

## Revendications

1. Microparticule solide, qui comprend des particules solides poreuses et une formule liquide non aqueuse,
la formule liquide non aqueuse comprend de 0,10 % à 4,00 % d'ingrédient pharmaceutique actif hydrophobe,
de 28,00 % à 99,90 % de solubiliseur liquide hydrophobe,
de 0 à 70,00 % de tensioactif non ionique, et
de 0 à 1,00 % d'antioxydant, les pourcentages font référence aux pourcentages en poids représentant la formule liquide non aqueuse ; dans laquelle le solubiliseur liquide hydrophobe comprend un mono-/diglycéride à chaîne moyenne et/ou un monoester d'acide gras de propylène glycol ;
le principe pharmaceutique actif hydrophobe est le dutastéride, et
les particules solides poreuses sont de l'hydrogénophosphate de calcium.

2. Microparticule solide selon la revendication 1, dans laquelle la teneur du principe pharmaceutique actif hydrophobe est de 0,10 % à 2,00 % ou de 0,18 % à 2,00 %, et les pourcentages font référence aux pourcentages en poids représentant la formule liquide non aqueuse ;
et/ou la teneur du solubiliseur liquide hydrophobe est de 32,50 % à 99,07 %, de 39,60 % à 99,01 %, ou de 49,50 % à 74,26 %, et les pourcentages font référence aux pourcentages en poids représentant la formule liquide non aqueuse ;
et/ou, lorsque la teneur du tensioactif non ionique n'est pas 0, la teneur du tensioactif non ionique est de 15,00 % à 66,67 %, de 24,51 % à 59,41 %, ou de 24,75 % à 49,50 %,
et les pourcentages font référence aux pourcentages en poids représentant la formule liquide non aqueuse ;
et/ou, lorsque la teneur de l'antioxydant n'est pas 0, la teneur de l'antioxydant est de 0,10 % à 1,00 % ou de 0,17 % à 0,83 %, et les pourcentages font référence aux pourcentages en poids représentant la formule liquide non aqueuse.

3. Microparticule solide selon la revendication 1 ou revendication 2, dans laquelle,
lorsque le solubiliseur liquide hydrophobe est un mono-/diglycéride à chaîne moyenne, la formule liquide non aqueuse comprend de 0,10 % à 2,00 % d'ingrédient pharmaceutique actif hydrophobe, de 97,00 % à 99,80 % de mono-/diglycéride à chaîne moyenne, et de 0,10 % à 1,00 % d'antioxydant, et les pourcentages font référence aux pourcentages en poids représentant la formule liquide non aqueuse ;
et/ou, lorsque le solubiliseur liquide hydrophobe est un monoester d'acide gras de propylène glycol, la formule liquide non aqueuse comprend de 0,10 % à 4,00 % d'ingrédient pharmaceutique actif hydrophobe, de 95,00 % à 99,90 % de monoester d'acide gras de propylène glycol, et de 0 à 1,00 % d'antioxydant, et les pourcentages font référence aux pourcentages en poids représentant la formule liquide non aqueuse ;
et/ou le rapport pondéral des particules solides poreuses sur la formule liquide non aqueuse est 2: 1 à 3: 1, préférablement 2: 1.

4. Microparticule solide selon au moins l'une des revendications 1 à 3, dans laquelle la microparticule solide comprend de 0,025 % à 3,00 % d'ingrédient pharmaceutique actif hydrophobe, de 5,00 % à 59,90 % de solubilisant liquide hydrophobe et de 0 à 45,00 % de tensioactif non ionique, de 0 à 0,60 % d'antioxydant et de 40,00 % à 75,00 % de particules solides poreuses, et les pourcentages font référence aux pourcentages en poids représentant la microparticule solide.

5. Microparticule solide selon la revendication 4, dans laquelle la teneur du principe pharmaceutique actif hydrophobe est de 0,10 % à 2,00 %, et les pourcentages font référence aux pourcentages en poids représentant la microparticule solide ;
et/ou la teneur du solubiliseur liquide hydrophobe est de 5,00 % à 39,90 %, de 11,50 % à 35,00 %, ou de 13,20 % à 33,00 %, et les pourcentages font référence aux pourcentages en poids représentant la microparticule solide ;
et/ou la teneur du tensioactif non ionique est de 0 à 40,00 %, de 4,50 % à 40,00 %, de 8,25 % à 19,80 %, ou de 8,25 % à 16,50 %, et les pourcentages font référence aux pourcentages en poids représentant la microparticule solide ;
et/ou la teneur de l'antioxydant est de 0,025 % à 0,60 %, et les pourcentages font référence aux pourcentages en poids représentant la microparticule solide ;
et/ou la teneur des particules solides poreuses est de 40,00 % à 75,00 % ou de 60,00 % à 70,00 %, et les pourcentages font référence aux pourcentages en poids représentant la microparticule solide.

6. Microparticule solide selon la revendication 4, dans laquelle,
lorsque le solubiliseur liquide hydrophobe est un mono-/diglycéride à chaîne moyenne, la teneur du principe pharmaceutique actif hydrophobe est de 0,025 % à 1,20 %, la teneur du mono-/diglycéride à chaîne moyenne est de 24,50 % à 59,90 %, et les pourcentages font référence aux pourcentages en poids représentant la microparticule solide ;
et/ou, lorsque le solubiliseur liquide hydrophobe est un monoester d'acide gras de propylène glycol, la teneur du principe pharmaceutique actif hydrophobe est de 0,025 % à 2,40 %, la teneur du monoester d'acide gras de propylène glycol est de 23,00 % à 59,90 %,
et les pourcentages font référence aux pourcentages en poids représentant la microparticule solide ;
et/ou la microparticule solide comprend de 0,0025 % à 3,00 % d'ingrédient pharmaceutique actif hydrophobe, de 5,00 % à 59,90 % de solubilisant liquide hydrophobe, de 0 à 45,00 % de tensioactif non ionique, de 0,025 % à 0,60 % d'antioxydant, et de 40,00 % à 75,00 % de particules solides poreuses, et les pourcentages font référence aux pourcentages en poids représentant la microparticule solide.

7. Microparticule solide selon au moins l'une des revendications 1 à 6, dans laquelle,
lorsque le solubiliseur liquide hydrophobe comprend un mono-/diglycéride à chaîne moyenne, le mono-/diglycéride à chaîne moyenne est préférablement un mono-/di-caprylate/caprate, et plus préférablement un mono-/di-glycéride caprylique ;
et/ou, lorsque le solubiliseur liquide hydrophobe comprend du monoester d'acide gras de propylène glycol, le monoester d'acide gras de propylène glycol est préférablement du monolaurate de propylène glycol de type II et/ou du monocaprylate de propylène glycol de type II, et plus préférablement du monocaprylate de propylène glycol de type II ;
et/ou le type du tensioactif non ionique est de type PEGylé et/ou de type polyol, préférablement de type PEGylé ; et le tensioactif non ionique de type PEGylé est préférablement de l'huile de ricin de polyoxyéthylène 35 et/ou des polyoxylglycérides de caprylocaproyl ; et le tensioactif non ionique de type polyol est préférablement du polysorbate 80 ;
et/ou l'antioxydant est le dibutylhydroxytoluène et/ou le butylhydroxyanisole, préférablement le dibutylhydroxytoluène ;
et/ou les particules solides poreuses présentent une superficie spécifique >30 m²/g, et
adsorbent au moins 0,40 mL/g de la formule liquide non aqueuse tout en maintenant simultanément la fluidité.

8. Microparticule solide selon la revendication 7, dans laquelle la microparticule solide comprend 0,33 % de dutastéride, 33,00 % de monocaprylate de propylène glycol de type II, et 66,67 % d'hydrogénophosphate de calcium, et les pourcentages font référence aux pourcentages en poids représentant la microparticule solide ;
ou la microparticule solide comprend 0,33 % de dutastéride, 24,75 % de monocaprylate de propylène glycol de type II, 8,25 % de polyoxylglycérides de caprylocaproyle et 66,67 % d'hydrogénophosphate de calcium, et les pourcentages font référence aux pourcentages en poids représentant la microparticule solide ;
ou la microparticule solide comprend 0,33 % de dutastéride, 16,50 % de monocaprylate de propylène glycol de type II, 16,50 % de polyoxylglycérides de caprylocaproyle et 66,67 % d'hydrogénophosphate de calcium, et les pourcentages font référence aux pourcentages en poids représentant la microparticule solide ;
ou la microparticule solide comprend 0,33 % de dutastéride, 13,20 % de monocaprylate de propylène glycol de type II, 19,80 % de polyoxylglycérides de caprylocaproyle et 66,67 % d'hydrogénophosphate de calcium, et les pourcentages font référence aux pourcentages en poids représentant la microparticule solide ;
ou la microparticule solide comprend 0,33 % de dutastéride, 24,75 % de monocaprylate de propylène glycol de type II, 8,25 % d'huile de ricin de polyoxyéthylène 35 et 66,67 % d'hydrogénophosphate de calcium, et les pourcentages font référence aux pourcentages en poids représentant la microparticule solide ;
ou la microparticule solide comprend 0,33 % de dutastéride, 16,50 % de monocaprylate de propylène glycol de type II, 16,50 % d'huile de ricin de polyoxyéthylène 35 et 66,67 % d'hydrogénophosphate de calcium, et les pourcentages font référence aux pourcentages en poids représentant la microparticule solide ;
ou la microparticule solide comprend 0,33 % de dutastéride, 13,20 % de monocaprylate de propylène glycol de type II, 19,80 % d'huile de ricin de polyoxyéthylène 35 et 66,67 % d'hydrogénophosphate de calcium, et les pourcentages font référence aux pourcentages en poids représentant la microparticule solide ;
ou la microparticule solide comprend 0,33 % de dutastéride, 24,50 % de monocaprylate de propylène glycol, 8,17 % d'huile de ricin de polyoxyéthylène 35, 0,33 % de dibutylhydroxytoluène et 66,67 % d'hydrogénophosphate de calcium, et les pourcentages font référence aux pourcentages en poids représentant la microparticule solide ;
ou la microparticule solide comprend 0,33 % de dutastéride, 16,34 % de monocaprylate de propylène glycol, 16,33 % d'huile de ricin de polyoxyéthylène 35, 0,33 % de dibutylhydroxytoluène et 66,67 % d'hydrogénophosphate de calcium, et les pourcentages font référence aux pourcentages en poids représentant la particule solide.

9. Microparticule solide selon la revendication 1, dans laquelle la teneur de la formule liquide non aqueuse est de 25,00 % à 60,00 %, et le pourcentage fait référence aux pourcentages en poids représentant la microparticule solide.

10. Microparticule solide selon la revendication 1, dans laquelle le mono-/diglycéride à chaîne moyenne est une chaîne ester formée par l'estérification du glycérol avec un ou deux acides gras en C₆-C₁₂ ;
ou le monoester d'acide gras de propylène glycol est une chaîne ester formée par estérification de propylène glycol avec un acide gras en C₆-C₁₂.

11. Microparticule solide selon la revendication 1, dans laquelle lorsque le solubiliseur liquide hydrophobe comprend un mono-/diglycéride à chaîne moyenne, la chaîne moyenne du mono-/diglycéride à chaîne moyenne est une chaîne d'acide gras en C₈-C₁₀ ;
ou, lorsque le solubiliseur liquide hydrophobe comprend un monoester d'acide gras de propylène glycol, le monoester d'acide gras de propylène glycol est une chaîne d'acides gras en C₈-C₁₂.

12. Procédé de préparation de la microparticule solide selon au moins l'une des revendications 1 à 11, qui comprend les étapes suivantes :
(1) le mélange du principe pharmaceutique actif avec le solubiliseur liquide hydrophobe pour obtenir une solution lorsque la microparticule solide ne comprend pas le tensioactif non ionique et l'antioxydant ;
ou le mélange du tensioactif non ionique et/ou de l'antioxydant, et du principe pharmaceutique actif et du solubiliseur liquide hydrophobe pour obtenir la solution A lorsque la microparticule solide comprend le tensioactif non ionique et/ou l'antioxydant ;
et (2) le mélange des particules solides poreuses avec la solution obtenue à l'étape (1) ;
ou le mélange des particules solides poreuses avec la solution A obtenue à l'étape (1).

13. Composition pharmaceutique comprenant la microparticule solide selon au moins l'une des revendications 1 à 11 et la tamsulosine.

14. Composition pharmaceutique selon la revendication 13, dans laquelle la composition pharmaceutique est sous forme de capsule dure ;
ou la tamsulosine est des granulés de tamsulosine à libération prolongée ou des granulés de chlorhydrate de tamsulosine à libération prolongée.

15. Microparticule solide selon au moins l'une des revendications 1 à 11 ou composition pharmaceutique selon au moins l'une des revendications 13 et 14 pour son utilisation comme médicament pour traiter l'hyperplasie prostatique bénigne, dans laquelle le principe pharmaceutique actif hydrophobe est le dutastéride.
